# EUROPEAN PATENT SPECIFICATION

(11) **EP 0 524 346 B1**
(45) Date of publication and mention of the grant of the patent: **17.06.1998**
(21) Application number: 91121476.5
(22) Date of filing: 14.12.1991
(51) Int. Cl.: A61K 7/06, A61K 7/40, A61K 7/00

(54) **Method for protecting hair against thermal degradation and to aid in hair styling**
Verfahren zum Schutz des Haares gegenüber thermischem Abbau und zur Haarformung
Méthode pour proteger les cheveux contre la dégradation thermique et l'aide de mise en forme des cheveux

(30) Priority: 23.07.1991 US 734594; 16.10.1991 US 778342
(43) Date of publication of application: 27.01.1993
(73) Proprietor: HELENE CURTIS, INC., Chicago Illinois 60610 (US)
(72) Inventor: Galleguillos, Ramiro, Glendale Heights, IL (US); Hoshowski, Myra, Addision, IL (US); Nandagiri, Arun, Libertyville, IL (US); Bhatt, Darshna, Schaumburg, IL (US)
(74) Representative: Griffiths, Helen Sarah

(56) References cited:
- EP-A- 0 379 082
- WO-A-89/04653
- US-A- 4 044 121
- US-A- 4 196 190
- US-A- 4 300 580
- PATENT ABSTRACTS OF JAPAN vol. 4, no. 166 (C-031)
- PATENT ABSTRACTS OF JAPAN vol. 6, no. 49 (C-096)
- PATENT ABSTRACTS OF JAPAN vol. 6, no. 49 (C-96)

## Description

### FIELD OF THE INVENTION

The present invention is directed to an aqueous or alcoholic composition containing a thermoplastic polymer that is applied onto hair prior to shaping the hair, to protect the hair against thermal damage, and to provide a hair styling aid useful in thermo-styling hair in a particular shape or configuration. More particularly, the present invention is directed to a hair composition having water and/or alcohol as a carrier material that functions both to protect the hair against thermal damage, and to aid in thermo-styling, containing a thermoplastic polymer having a glass transition temperature below about 120°C, preferably in the range of about 20°C to about 100°C and that, after application to the hair, can be repeatedly heated and softened up to at least about 200°C without thermal degradation of the hair or of the polymer to form the hair in a desired configuration, and the heating removed while the hair is in the desired configuration to allow the polymer to harden into a tough film that retains the hair in the desired configuration. After styling, the hair can be combed repeatedly while retaining the style since adjacent hairs are not substantially adhered together. The polymer can be shampooed out of the hair to avoid polymer build-up.

### BACKGROUND OF THE INVENTION AND PRIOR ART

Hair that has been exposed to heat from styling appliances, such as curling irons, becomes weakened, as indicated by tensile strength testing. Applying a particular polymer on the surface of the hair prior to exposing the hair to heat, protects the hair from heat's damaging effects, and particularly from loss of tensile strength. The polymers become more "rubber-like" at around the temperature that the hair is exposed to from a curling iron and thus flow to form a continuous film that "protects" the surface of the hair. After the polymer has been applied to the hair, much of the heat's energy is absorbed by the polymer coating thereby protecting the hair from damage.

Normal hair can be so fine and limp, and so lacking in body that the hair does not hold a hair set well. Furthermore, the hair can become even less bodied and can be weakened further as a result of being subjected to chemically active hair treatments, such as permanent waves and tints. Additionally, hair can be weakened even further by other contributing factors, such as bleaching by sun exposure and/or chlorinated swimming pool water.

Normal hair is usually hydrophobic. However, many of the chemically active hair treatments remove the natural hydrophobic components from the hair. As a result, as the hydrophobicity of the hair decreases, the relative porosity of the hair increases and the hair tends to absorb water and swell more readily. In such a weakened and porous state, the water-swollen hair is more vulnerable to stretching and breaking.

Hair setting is basically the process of shaping wet hair by the steps of stretching the hair by curling the hair, fixing the hair in place by drying, then combing to give the finishing touches to provide the desired hair style. In particular, the setting of wet hair can be accomplished by making flat curls from strands of hair and fixing the curls with hairpins to produce "pin curls". Similarly, the wet hair can be set by using any of a variety of rollers or curlers to mechanically fix the hair. In either case, the winding of the wet hair is followed by drying, either by ambient air drying, electric drying or hot air drying.

The inherent problem encountered in hair setting is the natural tendency of the hair to return to its natural shape. For example, the set hair returns to its natural shape almost immediately if moistened. Likewise, high humidity conditions accelerate the tendency of the hair to return to its natural shape. Therefore, intensive efforts have been directed toward providing a hair set with sufficient holding power to maintain the designed hair style until at least the next shampoo, and, therefore, giving the hair set a degree of permanency.

As indicated by the natural tendency of hair to return to its natural shape, hair is an elastic structure. As a result, the slight deformations in the hair structure resulting from setting the hair are completely reversible. However, the rate of return of the hair to its natural shape is dependent upon the method used to deform, or set, the hair. Hair sets performed with wet strands of hair being rolled tightly, either in curls around the finger or on curlers, followed by drying the hair and unrolling the curlers after drying, corresponds to the release of the hair from a deformation-causing load. The deformation, or set, obtained can last for several days, but the set will not be retained if the hair is wetted.

The observations of hair deformation and relaxation are related to physical and chemical changes in the protein structure level of hair. Sufficient stretching of the hair causes partial transformation of the α-keratin protein structure of the hair into the β-keratin protein structure of the hair. This structural transformation is accompanied by a shift in relative position of the polypeptide chains that is facilitated by water moistening the hair. The shift in position of the polypeptide chains therefore disrupts the ionic and hydrogen bonds in the hair. During the drying procedure, new ionic and hydrogen bonds are formed that block the return to the α-keratin protein structure of hair. Gradually, the new protein linkages give way, under natural forces, such that the hair returns to its original state and length. If the hair is moistened, the return to the α-keratin form is virtually immediate.

Therefore, investigators have sought to delay the combined action of natural forces and moisture that causes the hair to return to its original state by applying solutions containing naturally-occurring or synthetic polymers after the hair is shaped into a desired configuration. When applied to the shaped hair from aqueous or aqueous/alcoholic solutions (setting lotions), the polymers leave a film on the hair, after drying, to help maintain the hair in the previously shaped configuration. The polymeric film promotes cohesion and gives stability to the hair set to maintain the hold of the hair set. The principal objective of a setting lotion is to cover the previously styled hair with an invisible polymeric film that will give the styled hair a degree of rigidity and protect the hair style against wind and humidity.

Hair spray products act in a similar manner. The hair spray products are applied to wet and/or dry hair and contain a polymer, or mixtures of polymers, that remain fixed on the previously styled hair and affect the hair in various ways. For example, a "mechanical" effect is exerted on each individual hair. The film-forming polymers are used to provide a flexible sheath of polymeric film on the shaped hair after drying and, therefore, for mechanical reasons, retard the return of each individual hair to its natural shape. In addition, the polymeric film provides an overall stiffening of the hair and the hair strands are welded together, and the final hair style has better cohesion, therefore resisting the natural forces that return the hair to its natural shape. Finally, the polymeric film protects the hair from humidity. The ability of the polymeric film to attract and absorb water is preferably minimal, such that the polymeric film retards moisture uptake by hair and retards the return of the hair to the α-keratin hair protein structure.

The general principles of hair setting are thoroughly discussed by C. Zviak, in The Science of Hair Care, Marcel Dekker, pp. 149-181 (1986). Zviak reviews both the polymers used in hair setting products and the formulation principles used to produce a hair set product that provides such beneficial hair set properties as improved hair style hold, easy application and combing, quick drying and non-stickiness, good hair body and bounce, increased hair volume and gloss, and hydrophobicity. It is evident that in the formulation of any end-use hair styling product, some of these benefits must be sacrificed to some degree to achieve a competing benefit. Therefore, the formulation of hair set products has proved difficult, and, as a result, hair set products have been developed in a variety of product forms.

The prior art reveals that nonionic, cationic and anionic polymers have been used in hair set products, with the anionic polymers providing the best hair set results. However, anionic polymers also have disadvantages, such as high water solubility and, therefore, low hydrophobicity, and low substantivity to hair fibers, therefore, easy elimination from the hair by combing and brushing. As a result, investigators have continued to search for compounds and compositions that provide the primary benefit of an improved durability of the hair set. As previously mentioned, to overcome some of the inherent disadvantages of the polymers utilized to set the hair, hair set products are made available in diversified forms in an attempt to minimize the drawbacks of the particular polymer used in the formulation. For example, hair set products are available as plasticizing lotions, plasticizing gels, aerosol foams, all-purpose lotions, hair sprays, and holding lotions. In each case, however, the polymer is applied only to previously styled hair to help maintain the hair in the previously styled configuration.

U.S. Patent Nos. 3,250,682 and 4,635,654 teach hair setting methods and compositions using fixative resins. Neither patent teaches that repeated setting is possible or how to protect hair from thermal damage. Further, neither patent teaches compositions useful for setting hair and enabling the hair to be combed through after setting.

US-A-4300580 describes a composition for grooming the hair which comprises a linear polyester derived from at least one dicarboxylic acid, at least one diol and a -SO₃M group. The polymer can be used in an aerosol spray, such as a hair spray. Hair spray normally works by glueing the hairs together to hold them in position. Once the hairs are glued together, combing or brushing them becomes difficult or impossible.

WO-A-8904653 discloses a heat-activated hair treating composition which contains a cross-linked polyethylen oxyd polymer.

Patent abstracts of Japan, Vol. 6, No. 49 teach a hair dressing composition polymerized from vinyl monomer, sulfonic acid groups and a second vinyl monomer. Said document does not teach that the style can be reheated and styled into a different shape without damage to the polymer.

### SUMMARY OF THE INVENTION

1. In accordance with the present invention, a new and improved method of protecting hair against thermal damage, and thermally setting hair has been found, which comprises:
applying a composition comprising about 30% to about 99.9% by weight of a carrier selected from the group consisting of water, alcohol and combinations thereof and about 0.1% to about 20% by weight of a thermoplastic aryl-sulfonated polyester fixative resin having a glass transition temperature (Tg) less than about 120°C to the hair prior to substantially configuring the hair in a desired configuration in an amount low enough to prevent the aryl-sulfonated polyester from substantially adhering adjacent hairs together;
thereafter heating the hair with a thermal appliance to heat the applied aryl-sulfonated polyester to its glass transition temperature (Tg);
configuring the hair while the aryl-sulfonated polyester is at a temperature of at least Tg;
cooling the aryl-sulfonated polyester to a temperature below Tg while the hair is in the desired configuration to harden the aryl-sulfonated polyester in a shape complementary to the shape of the configured hair; and thereafter
combing the hair without substantial loss of set retention.

This process of heating and cooling the applied polymer to set the hair can be repeated many times to reconfigure the hair without degrading the polymer. Further, the hair can be combed or brushed without substantial loss of the imparted style since adjacent hairs are not glued to each other. Preferably, the polymer can be removed by shampooing to prevent buildup of polymer on the hair.

The composition used in the present invention includes a water-soluble or alcohol-soluble thermoplastic fixative polymer or resin in an amount of at least about 0.1% up to about 20% by weight of the composition, and preferably, in an amount in the range of about 0.5% to less than about 5% by weight of the composition, e.g. about 4% by weight, particularly about 0.5% to about 3% by weight of the composition. The fixative polymer should not be present in an amount that results in adjacent hairs being fixed to each other, or combing through after setting would adversely affect the set applied. Generally, a polymer amount of 5% by weight or more in the composition will adhere adjacent hairs together, although some resins having a Tg less than about 120°C can be applied from a composition that includes the resin in an amount up to about 10% by weight without adhering adjacent hairs together.

The methods of the present invention are surprisingly more effective than application of the same resins in the manner of a hair spray coating over configured hair, in a lower polymer percentage, since the polymers are thermally softened and, thereafter, hardened when the hair is in a desired configuration. The amount of water and/or alcohol carrier(s) in the compositions of the present invention can be in the range of about 0.5% to about 99.9% by weight of the composition; alcohol may be included in an amount of 0% to about 99.9% by weight; and, with aerosol compositions, a liquified propellant gas, such as dimethyl ether, is included in the compositions in an amount of about 5% to about 50% based on the total weight of the aerosol composition. Preferably, water is the predominant carrier and is present in an amount of about 30% to about 99.9% by weight of the composition. Alcohol, preferably is included in the composition in an amount of about 0.1% to about 30% by weight, and may be the sole carrier in an amount up to about 99.9% by weight of the composition.

Accordingly, one aspect of the present invention is to provide a new and improved hair treating method, that can be applied to the hair from an aqueous composition, prior to substantial configuring of the hair in a desired, final configuration, in the form of an aerosol or non-aerosol hair spray, mousse, foam or gel for preventing thermal damage to the hair, and for thermally retaining a particular shape or configuration of the hair. The hair then can be repeatedly combed or brushed since adjacent hairs are not substantially adhered together.

Another aspect of the present invention is to provide an aqueous hair styling aid method, using a composition in the form of a hair spray, mousse, foam or gel, that can be heat softened with common hair treating thermal appliances, such as curling irons, hot crimpers, heated rollers, hair dryers or other types of hair heating devices used for drying or shaping of hair; wherein the composition includes a thermoplastic polymer capable of hardening rapidly, while the hair is in a desired configuration, upon removal of the thermal appliance from the hair, to provide a durable hair set capable of repeated combing without substantial set loss.

Another aspect of the present invention is to provide a method of configuring or styling hair with a hair styling aid composition in the form of a hair spray, mousse, foam or gel that includes water in an amount of about 20% to about 99.9% by weight of the composition; and a thermoplastic polymer having a glass transition temperature below about 120°C, that is solubilized or dispersed in the water and/or alcohol carrier in an amount of about 0.1% to about 20%, preferably about 0.5% to about 3% by weight of the composition.

Still another aspect of the present invention is to use an improved aerosol or non-aerosol hair styling aid composition, that includes water in an amount of 0% to about 99.9%, preferably about 30% to about 95%, by weight of the composition; a thermoplastic fixative polymer, which may be a polyester containing a plurality of sulfonated aromatic dicarboxylic acid moieties, having a glass transition temperature less than about 120°C, and preferably less than about 100°C, that is solubilized in the carrier(s) in an amount of about 0.1% to about 20% by weight of the composition; alcohol in an amount of 0% to about 99.9%, preferably about 5% to about 25% by weight; and a propellant gas for aerosol compositions.

Still another aspect of the present invention is to use an improved aerosol or non-aerosol hair styling aid composition, that includes a carrier of water in a preferred amount of about 30% to about 99.9% by weight of the composition; a thermoplastic polymer having a glass transition temperature less than about 120°C, and preferably less than about 100°C, that is solubilized in the carrier in an amount of about 0.1% to about 20% by weight of the composition; alcohol in an amount of about 0% to about 30%, preferably about 5% to about 25% by weight alcohol as part of the carrier; a conditioning agent in an amount of about 0.1% to about 20% by weight of the composition, and a propellant gas for aerosol compositions.

The above and other aspects and advantages of the present invention will become more apparent from the following detailed description of the preferred embodiments, taken in conjunction with the accompanying drawings.

### BRIEF DESCRIPTION OF THE DRAWINGS

Figures 1 and 2 are graphs showing stress at break for hair treated with water (control) and hair treated with a composition containing 2% by weight of a thermoplastic polymer having a Tg less than 120°C (GAFFIX-713) with 0-3 heat treatments.

Figures 3, 4, 5 and 6 are graphs showing moisture regain of hair treated with water (control) and hair treated with a composition containing 2% by weight GAFFIX-713 with 0, 1 or 2 heat treatments.

### DETAILED DESCRIPTION OF THE PREFERRED EMBODIMENTS

The polymers or resins useful in the aqueous compositions of the present invention are
aryl-sulfonated polyesters, which can be rendered soluble by neutralization of pendant sulfonic acid groups, the general structural formula of which is as follows: 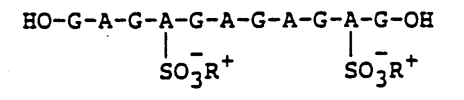
   - A =: an aromatic dicarboxylic acid moiety
   - G =: an aliphatic or cycloaliphatic glycol residue
   - OH =: hydroxy end groups
   - R =: H or a monovalent alkali metal, a primary, secondary or tertiary amine, ammonium, or an alkanolamine that have a glass transition temperature below about 120°C.

While the structural formula for the polymer shows two SO₃R substituents on dicarboxylic acid moieties of the polymer backbone, on average, there are five to eight SO₃R substituents per polymer molecule. The weight average molecular weight of the polymers generally is less than about 200,000 to provide a Tg less than about 120°C and, preferably, in the range of about 10,000 to about 50,000. The molecular weight, however, is not critical so long as the polymer has a Tg less than about 120°C.

To achieve the full advantage of these compounds, the fixative resin is an amorphous polyester that is dispersed or solubilized directly in water without the assistance of organic solvents, surfactants or amines.

Suitable polymers are sold by Eastman Corporation under the trademark EASTMAN AQ. EASTMAN AQ 29, for example, has a glass transition temperature of about 29°C. The dicarboxylic acid moieties have the general structural formula: 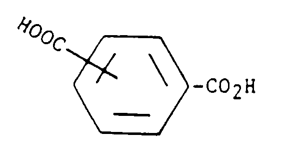
- e.g.,: phthalic acid (ortho);
isophthalic acid (meta); and
terephthalic acid (para).

Accordingly, the sulfonic acid and metal sulfonates of these dicarboxylic acid moieties have the following general structural formulas and both sulfonic acid moieties and the neutralized sulfonic acid moieties can be included together in the Polymer.

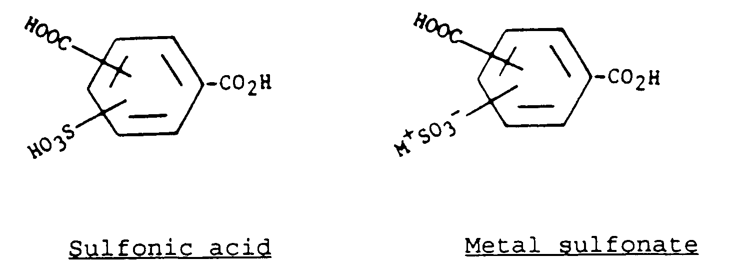

The aliphatic or cycloaliphatic glycol residues (G) have the following structural formulas: 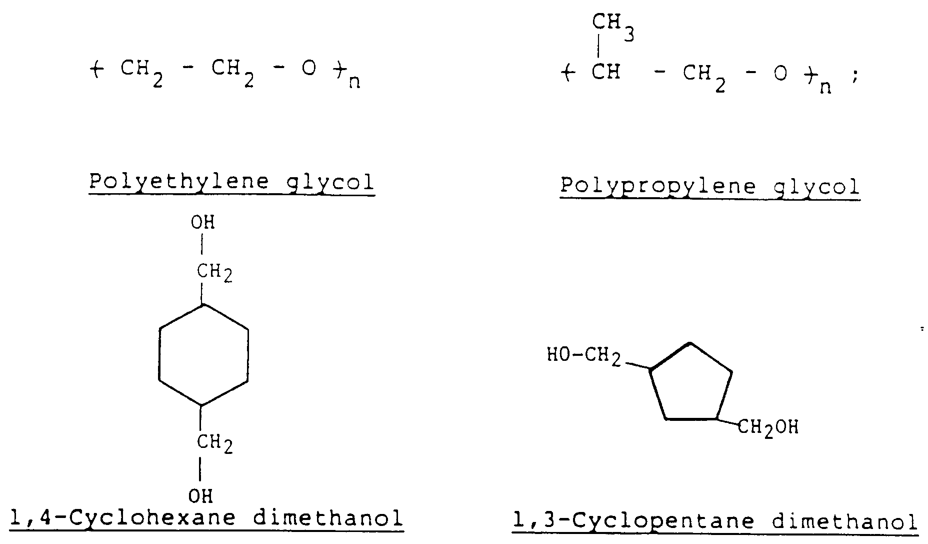 where n = 1 to about 200, preferably 1 to about 50.

Examples of the glycol residues are hexylene glycol; ethylene glycol; propylene glycol; 1,3 propane diol; 1,4 butane diol; 1,3 butane diol; 2,3 butane diol; 1,2-cyclopentane diol; 1,2-cyclohexane diol; pinacol; polyethylene glycol; polypropylene glycol, and the like.

Examples of these polymers include EASTMAN AQ Polymers 55, 38 and 29, which are water dispersible thermoplastic polymers including sodium sulfonate moieties. These are amorphous thermoplastic polyesters having good substantivity for hair. These polymers easily can be dispersed in warm and cold water, making them amenable for formulation and delivery from compressed aerosol and non-aerosol sprays, mousses, gels, lotions pomades, and the like, where the main carrier fluid is water, water/ethanol, water/isopropanol or water/water-soluble-glycol carrier mixtures. The amount of alcohol and/or glycol carrier that can be added to the solution depends on the type of resin. The tolerance decreases in the following order AQ 55< AQ 38 <AQ 29. For instance, it has been found that aqueous solutions of AQ 55 can tolerate no more than about 8% by weight of ethanol, i.e., the polymer starts to precipitate. Where polymer precipitation occurs at a higher alcohol and/or glycol percentage, compatible suspending, and/or stabilizing agents can be included. AQ 38 and 29 can tolerate up to about 20% ethanol. Since the polymers disperse in water and do not dissolve, their viscosity never exceeds more than about 100 centipoises at a 20% polymer concentration. Therefore, the amount of polymer that can be used in this invention is from about 0.1% to about 20% by weight. The polyester solutions/dispersions are stable at pH's between 1 and 12, although pH's between 3 and 9 are desired in personal care products. A number of adjuvants such as surfactants, emollients, silicones, monovalent mineral salts, fragrance, and the like can be added as desired.

The polymers useful in the compositions and methods of the present invention have relatively low glass transition temperatures below about 120°C, and preferably in the range of from about 20°C to about 60°C and preferably soften and flow at temperatures below 100°C. Calorimetric experiments such as differential scanning calorimetry (DSC) and thermogravimetric analysis (TG) have shown that the resins do not chemically degrade upon repeated heating and cooling. These polymers, therefore, are exceptionally suitable for thermo-styling of hair with curling irons, hot crimpers, rollers, and any other hot device used in hair styling. It has been have found that the temperature of most hot hair styling devices ranges from about 80°C to about 120°C.

Shaping of the hair is best accomplished by first applying the composition to hair while wet, allowing the hair to dry, and then physically shaping the hair with the hot styling aid. The heat softens the resin, thereby allowing it to spread along the hair shaft and acquire the configuration of the hot styling aid. After removing the hot styling aid, the resin hardens on individual hair shafts, maintaining the hair in the shape imparted by the styling aid. Because of the thermal stability of the resin, the hair can be restyled at any time, adding a convenience which is not possible to obtain with conventional hair fixative resins. Electron Scanning Microscopy (ESM) experiments on hair have shown that the main styling mechanism is not by gluing the hair fibers to one another, as is the case of conventional hair fixatives, but by coating the individual hair fibers with a hard, tough, thin film of resin which has enough mechanical strength to maintain the shape of the hair fiber. Coating of the hair fibers, and not gluing them together, also has the advantage of producing a durable hair style that easily can be combed through with less damage to hair.

Hair relaxation experiments have demonstrated that the shape of the hair (curls, waves, locks, zigzags, spirals, and the like) stays put until the hair is shampooed. Washability studies (optical and ESM) have shown that the resin can be washed out from the hair by simple shampooing. No resinous build-up was seen after five exaggerated cycles: treatment of hair with a 6% by weight solution, drying under air, hot curling for 30 seconds and washing with a solution of water and 10% sodium lauryl sulfate.

In accordance with another important feature of the present invention, any ionizable metal salt, wherein the metal has a valence of at least II, can be included in the composition, for polymer crosslinking, to improve the hair set retention properties of treated human hair. Hair treated with the aqueous thermoplastic polymer compositions of the present invention adjusted to a proper pH, e.g., above about 8.0, and containing an ionizable metal salt exhibits improved hair set retention properties if the metal of the ionizable metal salt has a valence of at least II.

The polymers can be crosslinked with polyvalent metal compounds such as those described in U.S. Patent No. 3,850,178, hereby incorporated by reference. Other useful polyvalent metal compounds include those described in U.S. Patent No. 4,036,241, at lower pH's, and this assignee's U.S. Patent No. 4,960,588, both patents hereby incorporated by reference. A crosslinked polymer composition, preferred in accordance with the present invention is obtained by first dispersing the resin in water, adding ammonium hydroxide to pH 9.0 or higher, then adding potassium or ammonium alum. The composition then is applied onto the hair while wet and the hair is allowed to dry.

When the fixative resin contains one or more sulfonic acid groups, as the hair dries, some of the ammonium hydroxide will be lost as ammonia gas, leaving sulfonic acid groups free to react with the alum-derived Al⁺³ atoms, as

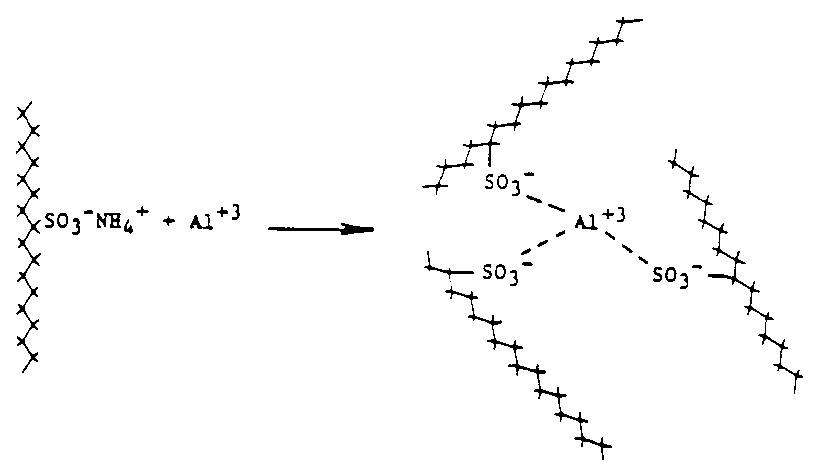

It is preferred to keep the level of surfactants as low as possible (preferably less than about 5% by weight and, more preferably, less than about 3% by weight) to avoid overplastization of the resin. Sandoperm FE or any other water-dispersible silicone, or other conditioning agent, can be added for conditioning benefits. Fragrance preferably is added last. This solution is placed in a mousse can or bottle and charged with propellant. Propellants can be any of the gases known in the art. It is preferred to use dimethyl ether (DME) or blends of DME with a hydrocarbon gas.

With certain of the polymers, it may be advantageous to neutralize some acid, e.g., sulfonic acid, moieties to promote solubility/dispersibility. Neutralization and increased solubilization are accomplished with one or more inorganic bases, such as sodium hydroxide, potassium hydroxide, ammonium hydroxide and/or ammonium carbonate. Among stable organic bases are the water soluble bases, such as monoethanolamide (MEA), diethanolamine (DEA), triethanolamide (TEA), 2-methyl-2-amino-1-propanol (AMP), monoamino glycols, and the like, which help solubilize the polymer in water solutions. The level of neutralization required for solubilization varies for each polymer. All of the above-described polymers become soluble or readily dispersible in water and hydroalcoholic solutions at 100% neutralization, and all described levels of water and alcohol solutions with or without a propellant. The pH of these solutions usually ranges from about 9 to about 12.

In accordance with one important embodiment, the composition of the present invention also includes from about 0.1% to about 10%, particularly about 0.5% to about 10%, and preferably from about 1.0% to about 5.0%, by weight of a non-volatile silicone compound or other conditioning agent(s), preferably a water-insoluble, emulsifiable conditioning agent. The preferred non-volatile silicone compound is a polydimethylsiloxane compound, such as a mixture, in about a 3:1 weight ratio, of a low molecular weight polydimethylsiloxane fluid and a higher molecular weight polydimethylsiloxane gum. The non-volatile polydimethylsiloxane compound is added to the composition of the present invention in an amount sufficient to provide improved combing and improved feel (softness) to the hair after shampooing. As referred to herein, "silicone gums" are those nonfunctional siloxanes having a viscosity of from about 5 to about 600,000 centistokes at 25°C. The so-called rigid silicones, as described in U.S. Patent No. 4,902,499, herein incorporated by reference, having a viscosity above 600,000 centistokes at 20°C, e.g., 700,000 centistokes plus, and a weight average molecular weight of at least about 500,000 also are useful in accordance with the present invention.

Preferred silicone gums include linear and branched polydimethylsiloxanes, of the following general formula:

(CH₃)₃SiO-[Si(CH₃)₂O]ₙ-Si(CH₃)₃ ,

wherein n is from about 2,000 to about 15,000, preferably from about 2,000 to about 7,000. Silicone gums useful in compositions of the present invention are available from a variety of commercial sources, including General Electric Company and Dow Corning.

Another particularly suitable conditioning agent that can be included in the composition of the present invention is a volatile hydrocarbon, such as a hydrocarbon including from about 10 to about 30 carbon atoms, that has sufficient volatility to slowly volatilize from the hair after application of the aerosol or non-aerosol styling aid composition. The volatile hydrocarbons provide essentially the same benefits as the silicone conditioning agents.

The preferred volatile hydrocarbon compound is an aliphatic hydrocarbon including from about 12 to about 24 carbon atoms, and having a boiling point in the range of from about 100°C to about 300°C. Exemplary volatile hydrocarbons are depicted in general structural formula (I), wherein n ranges from 2 to 5,

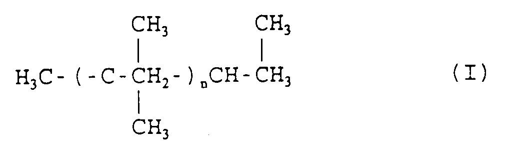

Examples of volatile hydrocarbons useful in the composition of the present invention are the commercially-available compounds PERMETHYL 99A and PERMETHYL 101A, corresponding to compounds of general structure (I) wherein n is 2 and 3, respectively, available from Permethyl Corporation, Frazer, PA. A volatile hydrocarbon compound is useful in the composition of the present invention either alone, in combination with another volatile hydrocarbon, or in combination with a volatile silicone.

Examples of other suitable water-insoluble conditioning agents that can be incorporated into the aerosol or non-aerosol aqueous styling aid composition of the present invention include the following: polysiloxane polyether copolymers; polysiloxane polydimethyl dimethylammonium acetate copolymers; acetylated lanolin alcohols; dimethyl dialkyl ammonium chlorides; modified alkyl dimethyl benzyl ammonium chlorides; lauryl dimethylamine oxide; stearyl dimethyl benzyl ammonium chloride; a lanolin-derived extract of sterol on sterol esters; lanolin alcohol concentrate; an isopropyl ester of lanolin fatty acids; sulfur rich amino acid concentrates; isopropyl ester of lanolin fatty acids; cetyl trimethyl ammonium chloride; oleyl dimethyl benzyl ammonium chloride; oleyl alcohol; stearyl alcohol; stearamidopropyl dimethyl myristyl acetate; a polyol fatty acid; a fatty amido amine; guar hydroxypropyltrimonium chloride; cetyl/stearyl alcohol; quaternized protein; keratin protein derivatives; isostearamidopropyl dimethylamine; stearamidopropyl dimethylamine; cetrimonium bromide; myrtrimonium bromide; stearalkonium chloride; cetyl trimethyl ammonium chloride; laurylpyridinium chloride; tris(oligoxyethyl)alkyl ammonium phosphate; an amino-functional silicone; lapyrium chloride; isopropyl ester of lanolic acids; ethoxylated (30) castor oil; acetylated lanolin alcohol; fatty alcohol fraction of lanolin; a mineral oil and lanolin alcohol mixture; high molecular weight esters of lanolin; quaternium-75; vinylpyrrolidone/dimethylaminoethylmethacrylate copolymer; alkyl trimethyl ammonium chloride; 5 mole ethylene oxide adduct of soya sterol; 10 mole ethylene oxide adduct of soya sterol; stearic acid ester of ethoxylated (20 mole) methyl glucoside; sodium salt of polyhydroxycarboxylic acid; hydroxylated lanolin; cocamidopropyl dimethylamine lactate; cocamidopropyl dimethylamine propionate; cocamidopropyl morpholine lactate; isostearamidopropyl dimethylamine lactate; isostearamidopropyl morpholine lactate; oleamidopropyl dimethylamine lactate; linoleamidopropyl dimethylamine lactate; stearamidopropyl dimethylamine lactate, ethylene glycol monostearate and propylene glycol mixture; stearamidopropyl dimethylamine lactate; acetamide MEA; lactamide MEA; stearamide MEA; behenalkonium chloride; behenyl trimethyl ammonium methosulfate and cetearyl alcohol mixture; cetearyl alcohol; isostearamidopropalkonium chloride; linoleamidopropalkonium chloride; oleyl dimethyl benzyl ammonium chloride; tallow imidazolinum methosulfate; stearyl trimonium methosulfate; mixed ethoxylated and propoxylated long chain alcohols; stearamidopropyl dimethylamine lactate; polonitomine oxide; oleamine oxide; stearamine oxide; soya ethyldimonium ethosulfate; hydroxypropyl bislauryl dimonium chloride; hydroxypropyl biscetyl dimonium chloride; hydroxypropyl bisstearyl dimonium chloride; hydroxypropyl bisbehenyl dimonium chloride; ricinolamidopropyl ethyldimonium ethosulfate; olealkonium chloride; stearalkonium chloride; N-(3-isostearamidopropyl)-N,N-dimethyl amino glycolate; N-(3-isostearamidopropyl)-N,N dimethyl amino gluconate; hydrolyzed animal keratin; ethyl hydrolyzed animal keratin; stearyl ammonium chloride; stearamidoethyl diethylamine; cocamidopropyl dimethylamine; lauramidopropyl dimethylamine; oleamidopropyl dimethylamine; palmitamidopropyl dimethylamine; stearamidopropyl dimethylamine lactate; avocado oil; sweet almond oil, grape seed oil; jojoba oil; apricot kernel oil; sesame oil; hybrid safflower oil; wheat germ oil; cocamidoamine lactate; ricinoleamido amine lactate; stearamido amine lactate; stearamido morpholine lactate; isostearamido amine lactate; isostearamido morpholine lactate; wheat germamido dimethylamine lactate; behenamidopropyl betaine; ricinoleamidopropyl betaine; wheat germamidopropyl dimethylamine oxide; disodium isostearaimido MEA sulfosuccinate; disodium oleamide PEG-2 sulfosuccinate; disodium oleamide MEA sulfosuccinate; disodium ricinoleyl MEA sulfosuccinate; disodium wheat germamido MEA sulfosuccinate; disodium wheat germamido PEG-2 sulfosuccinate; stearamido amine; stearamido morpholine; isostearamido amine; isostearamido morpholine; polyethylene glycol (400) mono and distearates; synthetic calcium silicate; isostearic alkanolamide; ethyl esters of hydrolyzed animal protein; blend of cetyl and stearyl alcohols with ethoxylated cetyl or stearyl alcohols; amido amines; polyamido amines; palmityl amido betaine; propoxylated (1-20 moles) lanolin alcohols; isostearamide DEA; and hydrolyzed collagen protein.

When one or more of these water-insoluble conditioning agents is included in the composition of the present invention in an amount of about 0.5% to about 10% by total weight of the composition, the composition also can include a suspending agent for the conditioning agent, in an amount of about 0.5% to about 10%, by total weight of the composition. The particular suspending agent is not critical and can be selected from any materials known to suspend water-insoluble liquids in shampoo compositions. Suitable suspending agents are for example, distearyl amate (distearyl phthalamic acid); fatty acid alkanolamides; esters of polyols and sugars; polyethylene glycols; the ethoxylated or propoxylated alkylphenols; ethoxylated or propoxylated fatty alcohols; and the condensation products of ethylene oxide with long chain amides. These suspending agents, as well as numerous others not cited herein, are well known in the art and are fully described in the literature, such as McCUTCHEON'S DETERGENTS AND EMULSIFIERS, 1989 Annual, published by McCutcheon Division, MC Publishing Co.

A nonionic, water-soluble alkanolamide also is optionally included in an amount of about 0.1% to about 5% by weight in the styling aid compositions that include a conditioning agent to provide exceptionally stable emulsification of water-insoluble conditioning agents and to aid in thickening and foam stability. Other useful suspending and thickening agents can be used instead of the alkanolamides such as sodium alginate; guar gum; xanthan gum; gum arabic; cellulose derivatives, such as methylcellulose, hydroxybutylcellulose, hydroxyethylcellulose, hydroxypropylcellulose and carboxymethylcellulose; and various synthetic polymeric thickeners, such as the polyacrylic acid derivatives. Suitable water-soluble alkanolamides include, but are not limited to, those known in the art of hair care formulations, such as cocamide monoethanolamide (MEA), cocamide diethanolamide (DEA), soyamide DEA, lauramide DEA, oleamide monoisopropylamide (MIPA), stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA and combinations thereof. Other suitable suspending agents are disclosed in Oh et al. Patent No. 4,704,272; Grote et al. Patent No. 4,741,855; and Bolich, Jr. et al. Patent No. 4,788,006, which patents are hereby incorporated by reference.

Emulsion stabilizers also may be used in compositions of the invention. Useful examples include, such compounds as polyethylene glycol, silicone copolyols, polyvinyl alcohol, sorbitan monostearate, oleth-2, sorbitan monolaurate, and nonionic block copolymers of ethylene oxide and propylene oxide such as those marketed by BASF Wyandotte under the name PLURONICS®. When present, such stabilizers comprise from about 0.05% to about 1%, preferably from about 0.1% to about 0.8%, by weight of the composition.

The propellant gas included in the aerosol forms of the compositions of the present invention can be any liquifiable gas conventionally used for aerosol containers. Examples of materials that are suitable for use as propellants are dimethyl ether, propane, n-butane and isobutane, and other water-soluble hydrocarbon gases used singly or admixed. Water-soluble gases such as dimethyl ether, carbon dioxide, and/or nitrous oxide also can be used to obtain aerosols having reduced flammability.

Other insoluble, compressed gases such as nitrogen, helium and fully-fluorinated oxetanes and oxepanes also are useful to deliver the compositions from aerosol containers. Other means of delivery of the above-described aqueous styling aid compositions include, pump sprayers, all forms of bag-in-can devices, in situ carbon dioxide (CO₂) generator systems, compressors, and the like.

The amount of the propellant gas is governed by normal factors well known in the aerosol art. For mousses, the level of propellant is generally from about 3% to about 30%, preferably from about 5% to about 15% of the total composition. If a propellant such as dimethyl ether utilizes a vapor pressure suppressant (e.g., trichlorethane or dichloromethane), for weight percentage calculations, the amount of suppressant is included as part of the propellant.

Other common cosmetic additives can be incorporated with the essential ingredients of the present invention, as long as the basic properties of the hair setting composition are not adversely affected. These additives include, but are not limited to, commonly used fragrances, dyes, opacifiers, pearlescing agents, foam stabilizers, preservatives, water softening agents, acids, bases, sequestering agents, buffers and the like; and will usually be present in weight percentages of less than about 1% each, and about 2% to about 5% in total. The composition vehicle, or carrier, is predominantly water but organic solvents also can be added to the composition in order to solubilize compounds that are not sufficiently soluble in water. Suitable solvents include those that do not react with the ionizable metal salt or the amino-containing compound such as the lower alcohols like ethanol and isopropanol; polyols like glycerol; glycols or glycol ethers, like 2-butoxyethanol, ethylene glycol, ethylene glycol monoethyl ether, propylene glycol and diethylene glycol monomethyl ether; and mixtures thereof. These solvents can be present in the hair setting composition of the present invention in an amount from about 1% to about 75% by weight and, in particular, from about 5% to about 50% by weight, relative to the total weight of the composition.

The compositions can be thickened, for example, with sodium alginate, gum arabic, cellulose derivatives such as methylcellulose, hydroxyethylcellulose, hydroxypropylmethylcellulose and carboxylmethyl-cellulose, and various polymeric thickeners, such as acrylic acid derivaties. It is also possible to use inorganic thickeners such as bentonite. These thickeners are preferably present in the amount from about 0.1% to about 10% by weight and, in particular, from about 0.5% to about 3% by weight, relative to the total weight of the composition.

The compositions also can include anionic, amphoteric or nonionic surfactants, to impart cleansing and/or emulsifying properties to the composition. Likewise, the compositions can contain other emulsifiers, fatty alcohols, humectants, cationic surfactants, such as cetrimonium chloride, and similar materials to provide conditioning properties, aesthetic properties and desirable physical properties to the composition.

For example, representative nonionic surfactants include esters or polyols and sugars; the polyethoxylated and/or polypropoxylated alkylphenols; the polyhydroxylated polyethers of fatty alcohols; and the condensation products of ethylene oxide with long chain mercaptans or long chain amides. Similarly, representative anionic surfactants include alkali metal salts, ammonium salts or salts of amines or amino alcohols of fatty acids such as oleic acid; of the sulfates of fatty alcohols, principally C₁₂-C₁₄ and C₁₆ fatty alcohols; of the sulfates of polyethoxylated fatty alcohols; the alkylbenzenesulfonates, such as those wherein the alkyl moiety has about 12 to about 22 carbon atoms; or the alkylarylpolyether sulfates and monoglyceride sulfates. All these nonionic and anionic surfactants, as well as numerous others not cited here, are well known in the art and are fully described in the literature.

The optional alcohol employed in the composition is an aliphatic straight or branched chain monohydric alcohol having 2 to about 4 carbon atoms. Isopropanol and especially ethanol are preferred. The concentration of the alcohol in the composition should be less than about 20% by weight, and surprisingly can be as low as 0%, preferably 0% to about 10% by weight and more preferably about 5% to about 10% by weight. Some alcohol, in an amount of about 2% to about 10% by weight provides faster drying of the styling aid after application to the hair.

Where polymer precipitation occurs at a higher alcohol and/or glycol percentage, compatible suspending stabilizing agents can be included. The polymer solutions/dispersions are stable at pH's between 1 and 12, although pH's between 3 and 9 are desired in personal care products. A number of adjuvants such as surfactants, emollients, silicones, monovalent mineral salts fragrance, can be added as desired.

### EXAMPLE 1 (Comparison Example)

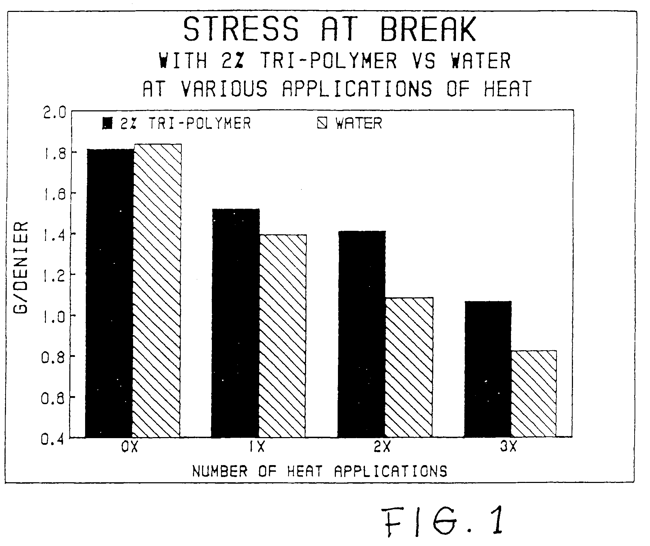

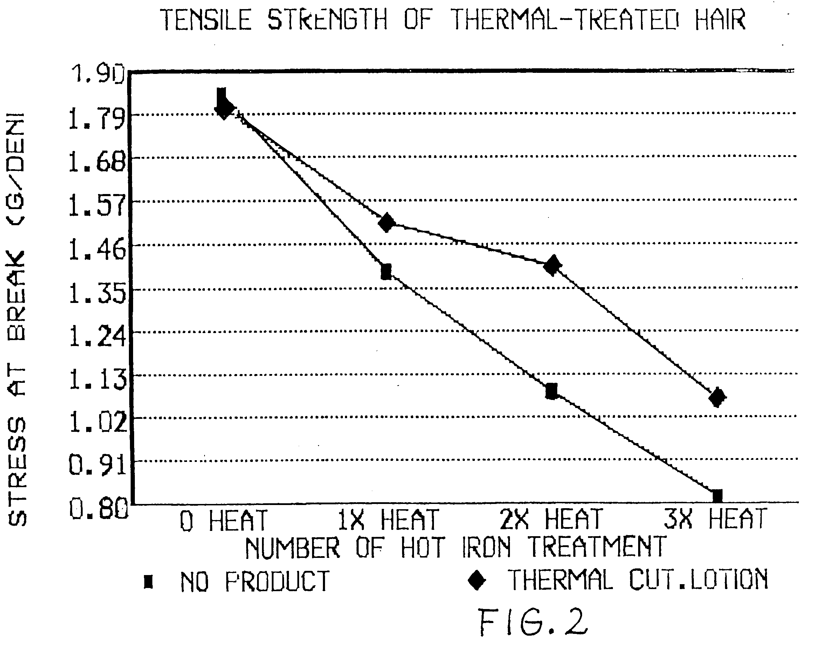

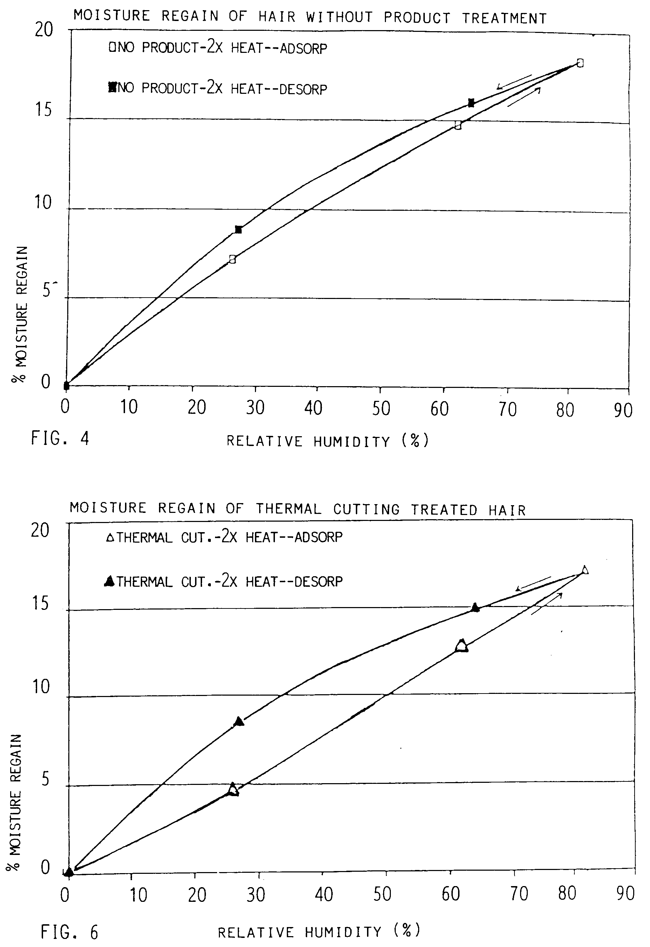

Clean, dry DeMeo brown hair was treated with the heat equivalent of a professional curling iron. Half the tresses did not have polymer on them (water only), the other half of the tresses have a water solution containing GAFFIX VC-713 -- a tri-polymer of vinylcaprolactam/polyvinyl pyrrolidone/ and dimethylaminoethyl methacrylate, in percentages ranging from 0.5% to 5% by weight in water. The heat was applied 0 times, 1, 2, and 3 times. The tresses are dried and equilibrated and the tensile strength was measured. The results that were obtained follow in Tables I and II and are also depicted in the graphs of Figures 1-6.

### Dry Tensile Test

The hair tresses were allowed to equilibrate at 30% relative humidity (RH), 21°C. The fibers were cut, weighed and mounted on self-adhesive paper tabs. The Dry Tensile Test was conducted at 60% RH, 25°C using the Instron Universal Testing Instrument, Series IX Software and computer. Briefly, an equilibrated test fiber was clamped between two grips and stretched at a uniform speed until it ruptured.

The tensile strength was assessed by the stress at break which is the load at rupture divided by the denier of the fiber (gram/denier). This parameter was measured in order to eliminate the effect of differences in fiber size. A higher value means better tensile strength than a lower value.

### Moisture Regain Test

The conventional moisture regain (the weight of water present as a percentage of the dry weight of hair) is based on the fact that the hair is hygroscopic and adsorbs water vapor from a moist atmosphere and loses it in a dry atmosphere. Because past work has shown that hot curling iron-treated hair usually requires a higher relative humidity to attain a given moisture regain, the moisture regain of polymer coated-thermal treated hair was compared with that of the control (water)-thermal treated hair.

For the Moisture Regain Test, the standard tresses of control-no heat, 2X control-heat, polymer coated-no heat and 2X polymer coated-heat were cut off from the tab and placed in weighing bottles. The hair samples were equilibrated overnight at the lowest RH, weighed, then equilibrated overnight at the next higher RH, weighed, equilibrated overnight at the next higher higher RH. The process was then reversed so that the hair samples were exposed to lower and lower relative humidities and then dried in a vacuum oven (95-100°C) for one hour to obtain the dry weight of the hair.

### Results and Discussion

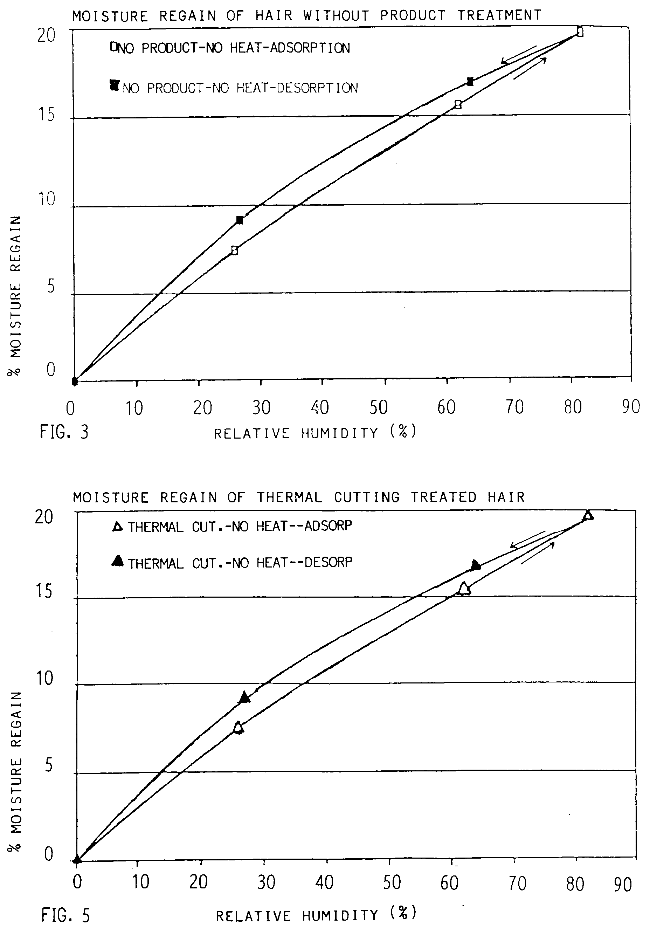

The test data were statistically analyzed using the Paired Differences T-Test and the findings are summarized in Tables I-II and graphically presented in Figures 3-6. All tresses were treated as uniformly as possible to avoid sample variability.

Briefly the results showed the following:
- In general, the dry tensile strength decreased with repeated thermal treatments.
- Pre-treatment with a thermoplastic polymer having a glass transition temperature less than 120°C retarded the heat damage--marginally in 1X thermal treated hair and significantly in 2X and 3X thermal treated hair.
- Moisture regain was lower in polymer coated-thermal treated hair than in the control-thermal treated hair.

The lower moisture regain results were not surprising in view of the fact that this test is known to be susceptible to pH, temperature, chemical treatment, and the like. This was, more or less, confirmed by the polymer coated-no heat samples which had been pre-treated with the polymer composition having a pH of 4.5-5.0 and showed marginal to significantly lower values than the control-no heat samples. If such hair regains less moisture, it should retain set curls better than hair without the polymer composition treatment.

**TABLE I**

| Stress at Break (grams/denier) Water vs. Polymer | | | | | | |
|---|---|---|---|---|---|---|
| # of Heat Applications | Water | | | 2% Vinylcaprolactam/PVP/DMAEMA | | |
| | Mean | | Std. Dev. | Mean | | Std. Dev. |
| 0 times | 1.8380 | ± | 0.1460 | 1.8120 | ± | 0.1857 |
| 1 time | 1.3923 | ± | 0.3591 | 1.5200 | ± | 0.4122 |
| 2 times | 1.0880 | ± | 0.2006 | 1.4100 | ± | 0.3220 |
| 3 times | 0.8243 | ± | 0.3230 | 1.0690 | ± | 0.3268 |

The statistical comparison using t-test showed that:

At 0 times heat, the water and polymer were not significantly different.

At 1 time heat, the polymer was significantly better than water at the 80% confidence level.

At 2 and 3 times heat, the polymer was significantly better than water at the 90% confidence level.

**TABLE II**

| MOISTURE REGAIN OF CONTROL AND POLYMER TREATED HAIR | | | | | | |
|---|---|---|---|---|---|---|
| | | 26% RH | 62% RH | 82% RH | 64% RH | 27% RH |
| | | % Moisture Regain | | | | |
| Control | Mean* | 7.480 | 15.577 | 19.646 | 16.898 | 9.170 |
| -No Heat | S.D. | 0.040 | 0.023 | 0.046 | 0.041 | 0.061 |
| 2X Control | Mean* | 7.116 | 14.762 | 18.369 | 15.995 | 8.872 |
| -Heat | S.D. | 0.051 | 0.064 | 0.100 | 0.073 | 0.080 |

| | | % Moisture Regain | | | | |
|---|---|---|---|---|---|---|
| Polymer | Mean* | 7.434 | 15.476 | 19.561 | 16.713 | 9.057 |
| -No Heat | S.D. | 0.087 | 0.073 | 0.108 | 0.107 | 0.109 |
| 2X Polymer | Mean* | 4.567 | 12.600 | 16.987 | 14.865 | 8.420 |
| -Heat | S.D. | 0.436 | 0.485 | 0.532 | 0.414 | 0.259 |

| | | % Moisture Regain | | | | |
|---|---|---|---|---|---|---|
| Control | Mean* | 7.480 | 15.577 | 19.646 | 16.898 | 9.170 |
| -No Heat | S.D. | 0.040 | 0.023 | 0.046 | 0.041 | 0.061 |
| Polymer | Mean* | 7.434 | 15.476 | 19.561 | 16.713 | 9.057 |
| -No Heat | S.D. | 0.087 | 0.073 | 0.108 | 0.107 | 0.108 |

| | | % Moisture Regain | | | | |
|---|---|---|---|---|---|---|
| 2X Control | Mean* | 7.116 | 14.762 | 18.369 | 15.995 | 8.872 |
| -Heat | S.D. | 0.051 | 0.064 | 0.100 | 0.073 | 0.080 |
| 2X Polymer | Mean* | 4.567 | 12.600 | 16.987 | 14.865 | 8.420 |
| -Heat | S.D. | 0.436 | 0.485 | 0.532 | 0.414 | 0.259 |

| | | | | | | |
|---|---|---|---|---|---|---|
| *** 4 samples tested** | | | | | | |

Other standard hair tresses were shampooed with the Attractions Revitalizing Shampoo (1828-49B). The tresses were then treated as follows:
1. Some of the wet tresses were treated with 0.5 mL/tress of the respective polymer-coating composition and after working the composition into the hair well, the tresses were allowed to air dry.
2. Both the control (water treated) and polymer-treated tresses were given a 15 sec. hot flat iron treatment (G.E. Travel Iron No. 12F38) on each side (front and back) of the tress.
3. All of the tresses were shampooed with the Attractions Revitalizing Shampoo.
4. The one-time polymer-treated tresses received another series of treatment (steps 1 and 2) while the control tresses were just heat-treated (step 2).

### Dry Tensile Test

The hair tresses were removed and allowed to equilibrate at 30% RH, 22°C. The fibers were cut, weighed and mounted on self-adhesive paper tabs. The Dry Tensile Test was conducted at 60% RH, 25°C using the Instron Universal Testing Instrument, Series IX Software and computer.

The parameter that was measured for assessing the tensile strength was the stress at break (load at rupture divided by the denier of the fiber). The strain at break was also measured to determine whether there was any change in the elongation of the fibers at break.

### Results and Discussion

Test data were statistically analyzed using an ANOVA and post hoc Tukey test. The findings are summarized in Table III.

Briefly, the results showed the following:
- All polymer treated hair (0.5-5% by weight polymer-containing compositions) had better dry tensile strength than the control hair (water treated-heat treated).
- Hair treated with the polymer compositions containing 5% by weight polymer demonstrated better tensile strength than those treated with compositions containing 0.5-3% by weight polymer. All polymer-treated tresses had similar tensile values.
- All polymer treated hair tresses elongated significantly more than the untreated control before rupturing.

The hair treated with the polymer composition containing 5% resin was significantly stronger than the other resin treated hair. One possible explanation may be the result of moisture regain. Tensile values decrease with increased moisture in hair, and visa versa. The 5% resin treated hair was considerably stiffer than the other resin treated hair and the polymer may have been applied in an amount sufficient to essentially seal the hair folicles against moisture uptake so that the hair regained less moisture at 60% RH, allowing it to behave like drier hair.

**TABLE III**

| DRY TENSILE PROPERTIES OF CONTROL AND POLYMER-TREATED HAIR | | | | |
|---|---|---|---|---|
| | Break Stress (gram/denier) | | Break Strain (%) | |
| | Mean* | S.D. | Mean* | S.D. |
| Untreated-No Heat | 1.8376 | 0.1460 | 47.8010 | 2.7866 |
| 2X 5% Resin-2X Heat | 1.6213 | 0.1287 | 44.4955 | 3.7146 |
| 2X .5% Resin-2X Heat | 1.5879 | 0.1950 | 41.9382 | 6.6035 |
| 2X 1% Resin-2X Heat | 1.5596 | 0.1188 | 42.7454 | 3.0740 |
| 2X 2% Resin-2X Heat | 1.4405 | 0.2713 | 40.8302 | 9.5138 |
| 2X 3% Resin-2X Heat | 1.4199 | 0.2170 | 40.6379 | 6.0358 |
| 2X Water-2X Heat | 1.0475 | 0.1658 | 33.2770 | 8.0102 |

| | | | | |
|---|---|---|---|---|
| *** 30 samples tested** | | | | |

A composition similar to the water solution of GAFFIX VC-713, but containing 22% alcohol, also was tested, at 2% and 3% by weight resin level, in comparison to a commercial product PAUL MITCHELL SEAL AND SHINE™ advertised to protect hair against excessive heat damage when applied to the hair prior to sculpting and blow drying, that contains sodium polystyrene sulfonate, having a Tg > 120°C. Also tested side-by-side was the composition containing 2% by weight resin containing no alcohol, and a water control. The break stress results of Table IV show a substantial advantage in the composition of the present invention including a fixative polymer having a Tg less than about 120°C.

**TABLE IV**

| DRY TENSILE PROPERTY OF NORMAL, BROWN HAIR | | |
|---|---|---|
| | Break Stress (gram/denier) | |
| | Mean* | S.D. |
| | | |

| (RELATIVE HUMIDITY 60% AND TEMPERATURE 25°C) | | |
|---|---|---|
| 3% Resin 22% Ethanol-2X Heat | 1.6581 | 0.01134 |
| 2% Resin (No Alcohol)-2X Heat | 1.4681 | 0.02292 |
| Paul Mitchell Shine and Seal 2X Heat - Tg > 120°C | 1.1733 | 0.0350 |
| Water Control Untreated Heated 2X | 1.0595 | 0.0449 |

| | | |
|---|---|---|
| *** n = 30 fibers** | | |

### Formulation Examples:

### EXAMPLE 2

### Hair Spray Concentrate:

| Item | Compound | | % Wt. |
|---|---|---|---|
| 1 | AQ 55 or 38 Polyester | = | 0.1 to 6% |
| 2 | Ethanol | = | 0 to 8% |
| 3 | Glycerine | = | 0 to 1% |
| 4 | Fragrance | = | as needed |
| 5 | Solubilizer | = | as needed |
| 6 | Water | = | Q.S. to 100% |

In formulating the composition of Example 1, item 1 is added first to water (item 6) until well dispersed. To this dispersion add items 2, 3, 4 and 5 in any order.

The above concentrate can be dispersed, as such, from a pump sprayer. It also can be used to make an aerosol product by charging the composition into an aerosol container with a suitable propellant gas, such as a hydrocarbon, a halogenated hydrocarbon, dimethyl ether (DME), carbon dioxide, compressed nitrogen, air, and the like. The preferred gas is DME in a composition (concentrate) to propellant ratio of about 65/35.

### EXAMPLE 3

### Mousse:

| Item | Compound | | % Wt. |
|---|---|---|---|
| 1 | AQ 55 or 38 | = | 0.1 to 6% |
| 2 | Anionic Surfactant | = | 0 to 0.5% |
| 3 | Nonionic Surfactant | = | 0 to 0.5% |
| 4 | Sandoperm FE | = | 0 to 2% |
| 5 | Fragrance | = | as needed |
| 6 | Propellant | = | 5 to 15% |
| 7 | Water | = | Q.S. to 100% |

In formulating the composition of Example 2, item 1 is first dispersed in Water (item 7). The surfactants (items 2 and 3) are added and dispersed well. It is preferred to keep the level of surfactants as low as possible (preferably less than about 5% by weight and, more preferably, less than about 3% by weight) to avoid overplastization of the resin. Sandoperm FE or any other water-dispersible silicone, or other conditioning agent, can be added for conditioning benefits. Fragrance preferably is added last. This solution is placed in a mousse can or bottle and charged with propellant. Propellants can be any of the gases known in the art. It is preferred to use the hydrocarbon gases, e.g., isobutane or a blend of 89% by weight isobutane with 16% by weight propane, e.g, with DME: Hydrocarbon = 3 to 7% by weight, and DME = 7 to 3% by weight. The mousse foam of this formulation is excellent, rich, creamy and stable and easy to apply to hair.

In formulating aqueous or hydroalcoholic aerosol formulations, the long term stability of the aerosol composition is important because it affects its shelf life, product performance and delivery. Unstable solutions will usually exhibit some kind of phase separation such as two livid phase formation or precipitation. Table I shows the results of stability tests at 110°F of Eastman AQ 38 aerosol solutions using various buffers, polymers, salts, surfactants, oils, acids, chelating and suspending agents. Most stable aerosol solutions that use DME as the propellant are prepared when the pH of the solution is maintained below about 5, preferably below about 3.5. This can be accomplished, for example, by adding any mineral or water-soluble carboxylic acid.

**TABLE V**

| Stability of 6% AQ 38 Aerosol Solutions C/P = 70/30. Propellant = DME | | | |
|---|---|---|---|
| Ingredient | Concentration % wt. | obs. at 110°F | pH |
| 6% AQ38/can | n/a | ppts/week | 6.805 |
| AMP | 1 drop | ppts/24hrs | 10.05 |
| Lavender Oil | 0.1 | ppts/24hrs | 6.70 |
| Oleth-2 | 0.1 | ppts/24hrs | 6.75 |
| Lav/Oleth-2 | 0.1/0.1 | ppts/24hrs | 6.74 |
| Prop. Glycol | 0.5 | ppts/24hrs | 6.82 |
| Dibutylene Glycol | 0.5 | ppts/24hrs | 6.73 |
| Finsolv TN | 0.5 | ppts/24hrs | 6.82 |
| Citric Acid | 0.5 | Stable | 2.72 |
| Buffer 7 | 6 | ppts/24hrs | 7.11 |
| Diethylene Glycol | 0.5 | ppts/24hrs | 6.79 |
| Tetrasodium EDTA | 0.2 | ppts/36hrs | 6.87 |
| Citric Acid | 0.01 | Stable | 4.47 |
| Na Acetate | 0.5 | ppts/36hrs | 6.49 |
| Amphomer | 4 | ppts/36hrs | 9.10 |
| Amphomer/NaCl | 3/0.5 | Stable/Hazy Mixture | 8.42 |
| NaCl/Citric Acid | 0.3/Q.A. to pH 4.9 | Stable Hazy | 4.95 |
| Citric Acid | Q.A. to pH 4.8 | Stable | 4.80 |
| AQ38/NaCl | 0.25 | Stable/Hazy | 6.10 |
| AQ38/NaCl | 1 | White milky soln with ppts | 6.10 |
| ppts = precipitate AMP = 2 amino - 2 methyl - 1 propanol Finsolv TM = is trade name by Finetex for C 12-13 alcohols benzoate Buffer 7 = is potassium phosphate monobasic sodium hydroxide buffer Amphomer = is trade name by National Starch for octyl acrylamide/acrylates/butyl amino ethyl methacrylate copolymer | | | |

## Claims

1. A method of protecting hair against thermal damage and thermally setting hair comprising:
applying a composition comprising about 30%to about 99.9% by weight of a carrier selected from the group consisting of water, alcohol and combinations thereof and about 0.1% to about 20% by weight of a thermoplastic aryl-sulfonated polyester fixative resin having a glass transition temperature (Tg) less than about 120°C to the hair prior to substantially configuring the hair in a desired configuration in an amount low enough to prevent the aryl-sulfonated polyester from substantially adhering adjacent hairs together;
thereafter heating the hair with a thermal appliance to heat the applied aryl-sulfonated polyester to its glass transition temperature (Tg);
configuring the hair while the aryl-sulfonated polyester is at a temperature of at least Tg;
cooling the aryl-sulfonated polyester to a temperature below Tg while the hair is in the desired configuration to harden the aryl-sulfonated polyester in a shape complementary to the shape of the configured hair; and thereafter
combing the hair without substantial loss of set retention.

2. The method of claim 1, wherein the polyester includes 5 to 8 SO₃R substituents per polymer molecule.

3. The method of claims 1 or 2, wherein the composition has a pH below about 3.5.

4. The method of any one of claims 1 to 3, wherein the thermoplastic aryl-sulfonated polyester is present in the composition in an amount of about 0.1% to about 10% preferably in an amount of about 0.1% to about 5.0%, in particular in an amount of about 0.1% to about 2.0% by weight of the composition.

5. The method of any one of claims 1 or 4, wherein the aryl-sulfonated polyester has a weight average molecular weight less than about 200,000.

6. The method of any one of claims 1 to 5, wherein the aryl-sulfonated polyester has a Tg in the range of about 20°C to about 80°C.

7. The method of of any one of claims 1 to 6, wherein the composition includes further about 1% to about 10% by weight of a hair conditioning agent and/or a surfactant in an amount less than about 5 %, preferably in an amount less than about 3 % by weight of the composition.

8. The method of any one of claims 1 to 7, wherein the composition includes further an ionizable metal salt, wherein the metal of the metal salt has a valence of at least II, in an amount sufficient to crosslink the sulfonate moieties of a plurality of aryl-sulfonated polyester molecules.

9. The method of any one of claims 1 to 8, wherein the hair treated with the aryl-sulfonated polyester is heated to a temperature in the range of about 80 °C to about 120 °C.

10. The method of any one of claims 1 to 9, wherein the aryl-sulfonated polyester composition includes a propellant gas and the aryl-sulfonated polyester is applied to the hair from an aerosol spray.

## Patentansprüche

1. Verfahren zum Schutz des Haares gegen thermische Schädigung und thermische Haarfestigung, umfassend:
Auftragen einer Zusammensetzung, umfassend etwa 30% bis etwa 99,9 Gew. -% eines Trägers, ausgewählt aus der Gruppe, bestehend aus Wasser, Alkohol und Kombinationen davon und etwa 0,1% bis etwa 20 Gew.-% eines thermoplastischen Aryl-sulfonierten Polyesterfixativharzes mit einer Glasübergangstemperatur (Tg) von weniger als etwa 120°C auf das Haar vor dem wesentlichen Konfigurieren des Haares in eine gewünschte Konfiguration in einer Menge, die gering genug ist, um den Aryl-sulfonierten Polyester vor wesentlichem Zusammenhaften benachbarter Haare zu bewahren;
anschließend Erhitzen des Haares mit einer thermischen Vorrichtung, um den aufgetragenen Aryl-sulfonierten Polyester auf dessen Glasübergangstemperatur (Tg) zu erhitzen;
Konfigurieren des Haares, während der Aryl-sulfonierte Polyester sich bei einer Temperatur von mindestens der Tg befindet;
Kühlen des Aryl-sulfonierten Polyesters auf eine Temperatur unterhalb der Tg, während sich das Haar in der gewünschten Konfiguration befindet, zum Härten des Aryl-sulfonierten Polyesters in einer Form, die komplementär zur Form des konfigurierten Haares ist; und anschließend
Kämmen des Haares ohne wesentlichen Verlust an Beibehalten der Festigung.

2. Verfahren nach Anspruch 1, wobei der Polyester 5 bis 8 SO₃R-Substituenten pro Polymermolekül einschließt.

3. Verfahren nach Ansprüchen 1 oder 2, wobei die Zusammensetzung einen pH-Wert unterhalb etwa 3,5 aufweist.

4. Verfahren nach einem der Ansprüche 1 bis 3, wobei der thermoplastische Aryl-sulfonierte Polyester in der Zusammensetzung in einer Menge von etwa 0,1% bis etwa 10%, vorzugsweise in einer Menge von etwa 0,1 % bis etwa 5,0%, insbesondere in einer Menge von etwa 0,1 % bis etwa 2,0 Gew.-%, der Zusammensetzung vorliegt.

5. Verfahren nach einem der Ansprüche 1 oder 4, wobei der Aryl-sulfonierte Polyester ein gewichtsmittleres Molekulargewicht von weniger als etwa 200 000 aufweist.

6. Verfahren nach einem der Ansprüche 1 bis 5, wobei der Aryl-sulfonierte Polyester eine Tg im Bereich von etwa 20°C bis etwa 80°C aufweist.

7. Verfahren nach einem der Ansprüche 1 bis 6, wobei die Zusammensetzung außerdem etwa 1% bis etwa 10 Gew.-% eines Haarkonditionierungsmittels und/oder ein Tensid in einer Menge von weniger als etwa 5%, vorzugsweise in einer Menge von weniger als etwa 3 Gew.-%, der Zusammensetzung, einschließt.

8. Verfahren nach einem der Ansprüche 1 bis 7, wobei die Zusammensetzung außerdem ein ionisierbares Metallsalz einschließt, wobei das Metall des Metallsalzes eine Wertigkeit von mindestens II aufweist, in einer Menge, die ausreicht, um die Sulfonatreste einer Vielzahl von Aryl-sulfonierten Polyestermolekülen zu vernetzen.

9. Verfahren nach einem der Ansprüche 1 bis 8, wobei das mit dem Aryl-sulfonierten Polyester behandelte Haar auf eine Temperatur im Bereich von etwa 80°C bis etwa 120°C erhitzt wird.

10. Verfahren nach einem der Ansprüche 1 bis 9, wobei die Aryl-sulfonierte Polyesterzusammensetzung ein Treibgas einschließt und der Aryl-sulfonierte Polyester aus einem Aerosolspray auf das Haar aufgetragen wird.

## Revendications

1. Procédé de protection des cheveux contre les atteintes thermiques et de mise en plis des cheveux dans lequel :
◆ on applique une composition comprenant d'environ 30% à environ 99,9% en poids d'un support choisi dans le groupe constitué par l'eau, l'alcool et leurs combinaisons et d'environ 0,1% à environ 20% en poids d'une résine fixative polyester aryl-sulfonée thermoplastique ayant une température de transition vitreuse (Tg) inférieure à environ 120°C aux cheveux avant de donner en pratique aux cheveux la configuration désirée, en une quantité suffisamment faible pour empêcher le polyester aryl-sulfoné de faire adhérer sensiblement entre eux les cheveux adjacents ;
◆ ensuite on chauffe les cheveux avec un appareil thermique pour chauffer le polyester aryl-sulfoné appliqué à sa température de transition vitreuse (Tg) ;
◆ on configure les cheveux tandis que le polyester aryl-sulfoné est à une température au moins égale à la Tg ;
◆ on refroidit le polyester aryl-sulfoné à une température inférieure à la Tg tandis que les cheveux sont dans la configuration désirée pour durcir le polyester aryl-sulfoné en une forme complémentaire de la forme des cheveux configurés ; puis
◆ on peigne les cheveux sans perte substantielle de rétention de la mise en plis.

2. Procédé selon la revendication 1, dans lequel le polyester inclut de 5 à 8 substituants SO₃R par molécule de polymère.

3. Procédé des revendication 1 ou 2, dans lequel la composition a un pH inférieur à environ 3,5.

4. Procédé de l'une quelconque des revendications 1 à 3, dans lequel le polyester aryl-sulfoné thermoplastique est présent dans la composition en une quantité d'environ 0,1% à environ 10%, de préférence en une quantité d'environ 0,1% à environ 5,0%, en particulier en une quantité d'environ 0,1% à environ 2,0% en poids de la composition.

5. Procédé de l'une quelconque des revendications 1 ou 4, dans lequel le polyester aryl-sulfoné a un poids moléculaire moyen en poids inférieur à environ 200 000.

6. Procédé de l'une quelconque des renvendications 1 à 5 dans lequel le polyester aryl-sulfoné a une Tg comprise entre environ 20°C et environ 80°C.

7. Procédé de l'une quelconque des revendications 1 à 6, dans lequel la composition inclut en outre d'environ 1% à environ 10% en poids d'un agent de conditionnement des cheveux et/ou d'un agent tensio-actif en une quantité intérieure à environ 5%, de préférence en une quantité inférieure à environ 3% en poids de la composition.

8. Procédé de l'une quelconque des revendications 1 à 7, dans lequel la composition inclut en outre un sel métallique ionisable, où le métal du sel métallique a une valence d'au moins II, en une quantité suffisante pour réticuler les parties sulfonates de plusieurs molécules de polyester aryl-sulfonées.

9. Procédé de l'une quelconque des revendications 1 à 8, dans lequel on chauffe les cheveux traités avec le polyester aryl-sulfoné à une température comprise entre environ 80°C et environ 120°C.

10. Procédé de l'une quelconque des revendications 1 à 9, dans lequel la composition de polyester aryl-sulfoné inclut un gaz vecteur, et l'on applique le polyester aryl-sulfoné aux cheveux à partir d'une pulvérisation d'aérosol.
